Europäisches Patentamt

(19) European Patent Office

Office européen des brevets

(11) Veröffentlichungsnummer: **0 088 971**
**B1**

(12) EUROPÄISCHE PATENTSCHRIFT

(45) Veröffentlichungstag der Patentschrift:
19.06.85

(51) Int. Cl.⁴: **C 12 M 1/00**

(21) Anmeldenummer: **83102184.5**

(22) Anmeldetag: **05.03.83**

(54) Vorrichtung zum Nachweis von Bakterien, Pilzen und Viren im Blut.

(30) Priorität: **13.03.82 DE 8207121 U**

(43) Veröffentlichungstag der Anmeldung:
**21.09.83 Patentblatt 83/38**

(45) Bekanntmachung des Hinweises auf die Patenterteilung:
**19.06.85 Patentblatt 85/25**

(84) Benannte Vertragsstaaten:
**AT BE CH DE FR GB IT LI LU NL SE**

(56) Entgegenhaltungen:
**BE - A - 859 687
DE - A - 2 857 361
FR - A - 2 282 277
FR - A - 2 436 607
GB - A - 1 441 022
GB - A - 1 525 177
US - A - 3 448 011**

(73) Patentinhaber: **Boehringer Mannheim GmbH,
Sandhoferstrasse 116, D-6800 Mannheim 31 (DE)**

(72) Erfinder: **Homann, Ernst, Lärchenweg 8,
D-6941 Laudenbach (DE)**
Erfinder: **Nelboeck, Michael, Dr. phil., Barelselweg 23,
D-8132 Tutzing (DE)**
Erfinder: **Schlieder, Klaus, Schwanenstrasse 65,
D-6800 Mannheim 51 (DE)**
Erfinder: **Bayer, Gernold, Speyerer Strasse 28,
D-6831 Brühl (DE)**

## Beschreibung

Die Erfindung betrifft eine Vorrichtung zum Nachweis von Bakterien, Pilzen und Viren im Blut mit einem Gehäuse, in dem sich ein die Bakterien, Pilze und Viren bindendes aus mehreren zur anschließenden bakteriologischen, virologischen, mykologischen oder elektronenmikroskopischen Untersuchung geeigneten Teilen bestehendes Adsorbens befindet, wobei das Gehäuse zwei Anschlüsse für Schlauchleitungen zum Zuführen und Abführen von in einem extrakorporalen Kreislauf fließendem Blut aufweist und wobei das Gehäuse im wesentlichen als Zylinder mit vorzugsweise kreisförmigem Querschnitt ausgebildet ist.

Eine derartige Vorrichtung, die im folgenden auch als diagnostisches Modul bezeichnet wird, ist aus der deutschen Patentschrift 2 826 416 bekannt. Sie dient insbesondere zum Erkennen septischer Komplikationen bei Patienten, die wegen einer anderweitigen schweren Erkrankung auf einer Intensivstation liegen. Wie in der zitierten Druckschrift ausführlich beschrieben wird, kommt es in diesen Fällen wesentlich darauf an, daß eine etwaige Infektion durch Bakterien, Viren oder Pilze möglichst schnell erkannt und der Erreger identifiziert wird, um ihn insbesondere mit einem spezifischen Antibioticum möglichst schnell bekämpfen zu können.

Vor der der zitierten Patentschrift zugrundeliegenden Erfindung wurde bei Verdacht einer Sepsis üblicherweise eine Blutkulturtechnik verwendet. Dabei war die Wahrscheinlichkeit, den Erreger zu identifizieren, mit ca. 30% sehr niedrig. Außerdem ist die Blutkulturtechnik insofern unbefriedigend, als zwischen der Blutentnahme und dem Befund eine zu lange Zeit vergeht.

Hier brachte nun die in der DE-PS 2 826 416 beschriebene Erfindung einen entscheidenden Fortschritt. Diese betrifft eine Vorrichtung, die im wesentlichen aus einem Gehäuse mit zwei Anschlüssen besteht, das vom Blut des Patienten in einem extrakorporalen Kreislauf durchströmt wird. In diesem Gehäuse befindet sich ein für therapeutische Zwecke an sich bekanntes Adsorbens. Die Erfinder dieser Vorrichtung haben nun erkannt, daß Bakterien, Pilze und Viren auf der Oberfläche dieses Adsorbens angereichert werden, wenn dieses von Blut durchströmt wird und daß sich diese Eigenschaft vorteilhaft für diagnostische Zwecke verwenden läßt. Um die Krankheitserreger auf dem Adsorbens anzureichern, wird der extrakorporale Kreislauf für beispielsweise 30 bis 60 Minuten aufrechterhalten, danach wird das diagnostische Modul abgetrennt, sodann beispielsweise mit physiologischer Kochsalzlösung durchspült und anschließend mit einem Nährmedium, einer sogenannten Nährbouillon, gefüllt und vorzugsweise bei Körpertemperatur bebrütet.

Danach werden Teilmengen der Nährlösung und des Adsorbens aus dem Gehäuse entnommen, das zu diesem Zweck mit mindestens einem abnehmbaren Deckel versehen ist. Bevorzugt liegt das Adsorbens in Form einer Vielzahl kleiner Festkörper als Granulat oder dergleichen vor, wodurch sich eine große adsorbierende Oberfläche ergibt. Da sich diese große Oberfläche aber auch durch eine geeignete poröse Struktur des Adsorbens erreichen läßt, kann dieses auch aus größeren Teilen, beispielsweise in Form von runden Scheiben bestehen. Diese Teile müssen jedenfalls so beschaffen sein, daß sie sich zur nachfolgenden mikrobiologischen Untersuchung eignen.

Um diese durchzuführen, werden entsprechende Teilmengen der Nährlösung und des Adsorbens bei der bekannten Vorrichtung aus dem Gehäuse, beispielsweise unter Zuhilfenahme eines Spatellöffels entnommen und auf einen Agar-Nährboden aufgebracht. Auf diesem werden dann die im Blut des Patienten vorhandenen und auf der Oberfläche des Adsorbens angereicherten oder in der Nährlösung vorhandenen Keime gezüchtet und später nach einem der bekannten mikrobiologischen Verfahren identifiziert. Weitere Einzelheiten sind der zitierten deutschen Patentschrift 2 826 416 zu entnehmen, auf die hier Bezug genommen wird.

Zusammengefaßt zeichnet sich das in der deutschen Patentschrift 2 826 416 beschriebene Verfahren gegenüber der zuvor üblichen Blutkulturmethode durch folgende Vorteile aus:

1) Durch die Anreicherung der Keime auf dem Adsorbens wird die Wahrscheinlichkeit eines positiven Nachweises von Keimen erhöht. Dies gilt insbesondere dann, wenn, wie bei Intensivpatienten sehr häufig der Fall, bereits Breitbandantibiotica eingesetzt worden sind. Es wurde beobachtet, daß diese in der Nähe der Oberfläche des Adsorbens in relativ verringerter Konzentration gefunden werden, so daß die Chance einer erfolgreichen Anzüchtung der dort angereicherten Keime gesteigert ist.

2) Die Gefahr eines falschpositiven Befundes bzw. der Identifizierung falscher Keime durch Verunreinigung wird vermindert. Dabei spielt die Tatsache eine erhebliche Rolle, daß wegen der Anreicherung der Keime keine extrem empfindlichen mikrobiologischen Nachweismethoden eingesetzt werden müssen, bei denen die Gefahr eines aus einer Verunreinigung stammenden falschpositiven Befundes besonders hoch ist.

3) In vielen Fällen läßt sich die Nachweisgeschwindigkeit erhöhen, dadurch daß die Keimanreicherung eine Beschleunigung des mikrobiologischen Nachweises erlaubt.

Trotz dieser entscheidenden Vorteile hat sich das Verfahren der DE-PS 2 826 416 noch nicht im wünschenswerten Maße durchsetzen können, weil den bisher bekannten diagnostischen Modulen im praktischen Einsatz wesentliche Nachteile zu eignen sind. Diese betreffen insbesondere die Entnahme der Adsorbensteile aus dem Gehäuse, die bisher mittels eines Spatels erfolgte, wobei die Dosierung auf den Agar-Platten verhältnismäßig schwierig und langwierig ist und eine erhebliche Verunreinigungsgefahr besteht. Dabei ergeben sich insbesondere Schwierigkeiten, weil die Adsorbensanteile nach dem Blutdurchgang häufig relativ fest aneinander haften.

Der Erfindung liegt daher die Aufgabe zugrunde, einen diagnostischen Modul gemäß DE-PS 2 826 416 dahingehend zu verbessern, daß eine vereinfachte Handhabung bei verminderter Verunreinigungsgefahr möglich ist. Dabei soll der Modul so gestaltet sein, daß er möglichst als Wegwerfteil einfach und kostengünstig herzustellen und in steriler Weise für den Einsatzzweck bereitzuhalten ist.

Diese Aufgabe wird bei einer Vorrichtung der eingangs näher bezeichneten Art dadurch gelöst, daß ein mittels einer aus dem Gehäuse herausragenden Kolbenstange betätigbarer Kolben zwischen einer ersten Position und einer zweiten Position gleitbar in dem Gehäuse angeordnet ist und daß in der ersten Position durch den Kolben, die zylindrische Gehäusewandung und einen abnehmbaren Deckel ein Hohlraum begrenzt wird, in dem sich das Adsorbens während des Anschlusses der Vorrichtung an einen extrakorporalen Kreislauf befindet und daß in der zweiten Position der Hohlraum kleiner ist als in der ersten Position, so daß bei der Bewegung des Kolbens von der ersten Position in die zweite Position bei abgenommenem Deckel mindestens ein Teil des Adsorbens aus dem Gehäuse herausgedrückt wird.

Gemäß einer bevorzugten Ausführungsform ist dabei einer der Anschlüsse für die blutführenden Schlauchleitungen ein Bestandteil der Kolbenstange und die Kolbenstange hat eine Längsbohrung, durch die das Blut strömen kann. In diesem Fall ähnelt die erfindungsgemäße Vorrichtung einer Spritze mit einer durchbohrten Kolbenstange, wobei diese Spritze in ihrer Gesamtheit für das Blut durchgängig ist, während der Kolben andererseits geeignet ist, die Adsorbensfüllung aus der Spritze herauszudrücken. Zu diesem Zweck weist er ein Bauteil auf, das man als Filter bezeichnen kann und das als Sperrelement für die Adsorbensfüllung wirkt. Wenn diese aus verhältnismäßig großen Teilen besteht, kann das Sperrelement aus einem oder mehreren Löchern bestehen, die sich im Kolbenboden befinden und kleiner als die kleinsten Teile des Adsorbens sind. Wenn das Adsorbens in bevorzugter Art und Weise als verhältnismäßig feines Granulat ausgebildet ist, besteht das Sperrelement bevorzugt aus einem geeigneten Sieb oder einer Sperrplatte mit Kanälen für das Blut.

Im Folgenden werden die Erfindung und die mit ihr zu erreichenden Vorteile anhand eines bevorzugten Ausführungsbeispiels näher erläutert, das in den Figuren dargestellt ist. Es zeigt

Fig. 1 die zentrale Einheit eines erfindungsgemäßen diagnostischen Moduls im Querschnitt,

Fig. 2 eine Aufsicht auf einen schematisch dargestellten bevorzugten diagnostischen Modul gemäß der Erfindung,

Fig. 3 eine Skizze zur Verdeutlichung der Handhabung des Moduls und

Fig. 4 eine Detailzeichnung einer bevorzugten Ausführungsform.

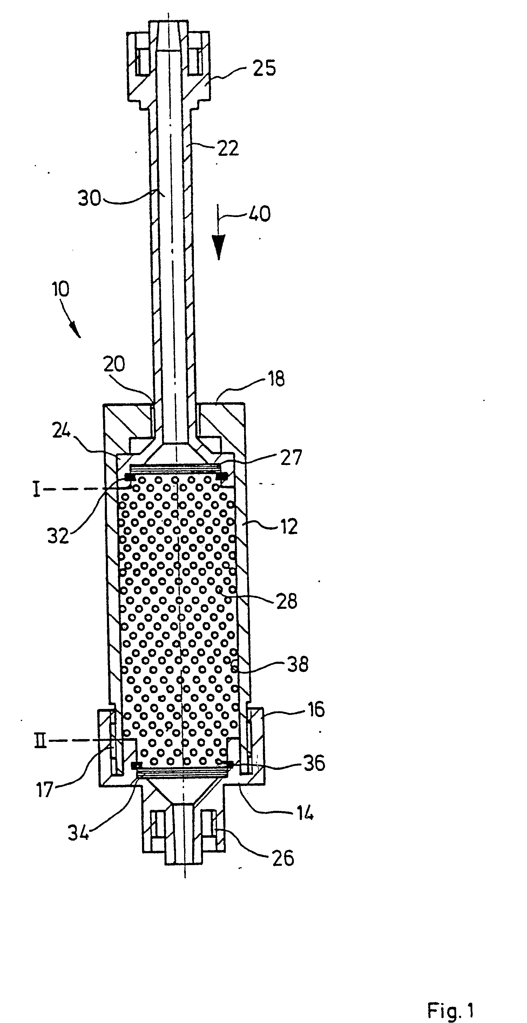

In Fig. 1 erkennt man einen in seiner Gesamtheit mit dem Bezugszeichen 10 bezeichneten diagnostischen Modul. Er besteht im wesentlichen aus einem zylinderförmigen Gehäuse 12, dessen in der Zeichnung unteres Ende 14 durch einen Deckel 16 verschlossen ist, der auf ein Gewinde 17 aufgeschraubt ist.

Das in der Zeichnung obere Ende 18 des Gehäuses 12 ist geschlossen bis auf eine Bohrung 20, die durch die Kolbenstange 22 des Kolbens 24 durchdrungen wird.

An dem vom Gehäuse 12 abgewandten Ende der Kolbenstange 22 befindet sich ein erster Anschluß 25 für Schlauchleitungen, die das Blut des Patienten in einem extrakorporalen Kreislauf transportieren. Ein zweiter derartiger Anschluß 26 befindet sich an dem Deckel 16. In beiden Fällen handelt es sich bevorzugt um sogenannte Luerlock-Anschlüsse, wie sie in der Medizintechnik üblich sind. In dem Kolben 24 befindet sich ein Sieb 27, das als Trennelement oder Filter wirkt, d. h. es verhindert, daß das Adsorbens 28 aus dem Hohlraum des zylinderförmigen Gehäuses 12 in die Bohrung 30 der Kolbenstange 22 gelangen kann. Das Trennelement 27 ist beispielsweise durch einen Klemmring 32 mit dem Kolben 24 verbunden.

In entsprechender Weise befindet sich in dem Deckel 16 ein Trennelement 34 und ein Klemmring 36.

In der Fig. 1 befindet sich der Kolben 24 in einer mit I bezeichneten ersten Position. Wie der Kolben einer Spritze ist er so gestaltet, daß er entlang der Innenwandung 38 des zylinderförmigen Gehäuses 12 in der Zeichnung von oben nach unten gleiten kann, wenn ein entsprechender Druck in Richtung des Pfeiles 40 auf die Kolbenstange 22 ausgeübt wird.

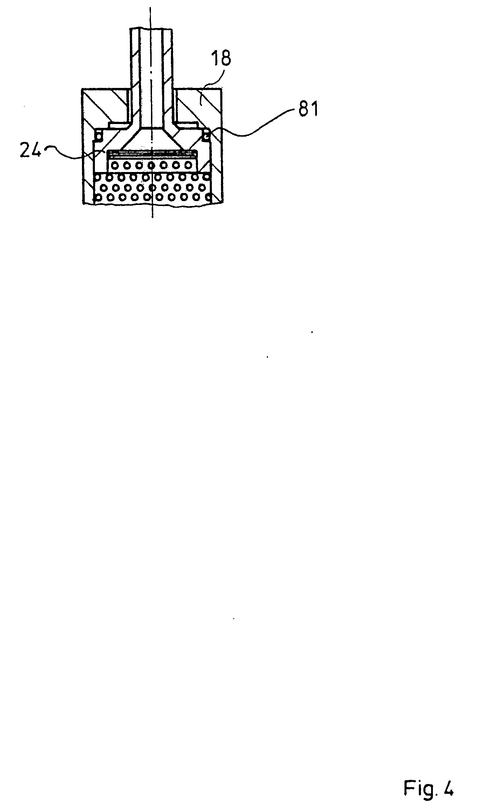

In einer weiteren bevorzugten Ausführungsform gemäß Fig. 4 ist der Kolben 24 konisch oder stufenförmig ausgebildet, wobei sein Durchmesser in Richtung der Kolbenstange 22 geringer ist als in Richtung des Deckels 16. Über die konische Ausbildung gezogen oder in die Stufe des Kolbens eingelegt, befindet sich ein O-Ring 81, der beim Verschieben des Kolbens von I nach II irreversibel vom Kolben rutscht und damit anzeigt, daß der Kolben bewegt wurde. Der Vorteil dieser Ausführungsform liegt darin, daß der Benutzer anhand der Position des O-Ringes 81 leicht feststellen kann, ob die erfindungsgemäße Vorrichtung benutzt worden ist und damit Teile des Adsorbens bereits herausgedrückt worden sind oder ob es sich noch um eine sterile, unbenutzte Vorrichtung handelt.

Zum Nachweis von Bakterien Pilzen und Viren im Blut eines Patienten wird die in Fig. 1 dargestellte Vorrichtung an einen extrakorporalen Kreislauf angeschlossen, so daß das Blut von dem ersten Anschluß 25 durch die Bohrung 30, das Trennelement 27, das Adsorbens 28, das Trennelement 34 und den Anschluß 26 fließt. Dieser Kreislauf wird, wie erwähnt, für einige Zeit, beispielsweise 30 bis 60 Minuten, aufrechterhalten. Dabei sammeln sich etwa in dem Blut befindliche Keime auf der Oberfläche der Adsorbensteile 28 an.

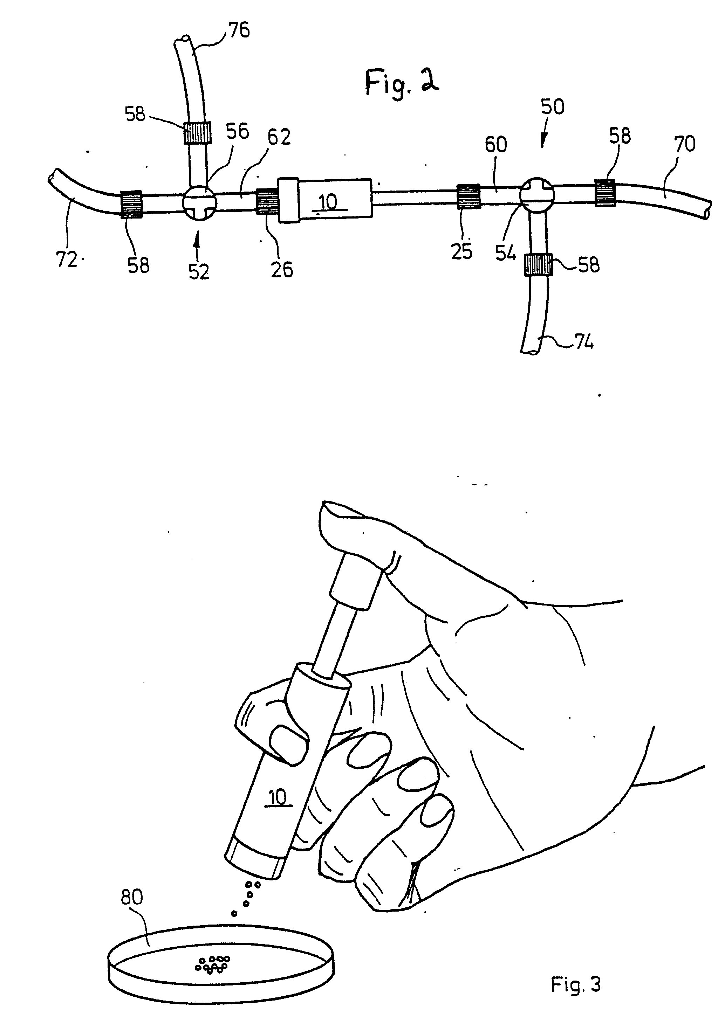

Danach muß das Gehäuse durchgespült und bebrütet werden. Zu diesem Zweck wird bevorzugt eine schematisch in Fig. 2 dargestellte Vorrichtung verwendet, bei der der diagnostische Modul 10 beidseitig mit einer Dreiwegverzweigung 50 bzw. 52 versehen ist, die im dargestellten Ausführungsbeispiel jeweils aus einem als Absperreinrichtung wirkenden Dreiwegehahn 54 bzw. 56 und entsprechenden Schlauchanschlußelementen 58 bestehen,

Die Dreiwegehähne 54 und 56 sind über Schlauchstücke 60 und 62 mit den Anschlüssen 25 und 26 des diagnostischen Moduls 10 verbunden. Prinzipiell können sie auch einstückig mit dem Deckel 16 bzw. mit der Kolbenstange 22 verbunden sein, jedoch hat dies fertigungstechnische Nachteile.

In Fig. 2 dienen die Schlauchleitungen 70 und 72 dem Anschluß des Blutkreislaufes. Bei der dargestellten Einstellung der Dreiweghähne 54 und 56 durchströmt das Blut die Vorrichtung von der Schlauchleitung 70 bis zur Schlauchleitung 72. Der besondere Vorteil der in Fig. 2 dargestellten Ausführungsform besteht nun darin, daß auf besonders einfache Weise nach Ablauf der für die Anreicherung der gesuchten Bakterien, Pilze oder Viren auf dem Adsorbens notwendigen Zeit, lediglich die Dreiweghähne 54 und 56 in eine Stellung gebracht werden müssen, bei denen die Schlauchleitungen 70 und 72 von dem diagnostischen Modul 10 abgetrennt sind und stattdessen die Leitungen 74 und 76 mit dem Inneren des diagnostischen Moduls 10 in Verbindung stehen. Diese Umschaltung ist bei der bevorzugten Ausführungsform der Erfindung möglich, ohne daß eine Kontaminationsgefahr besteht.

Nun kann beispielsweise durch die Leitung 74 ein Spülmittelstrom zugeführt werden, der durch die Leitung 76 abfließt. Danach wird über die gleichen Leitungen der diagnostische Modul 10 mit einer Nährbouillon beschickt.

Durch entsprechendes Weiterdrehen der Dreiweghähne 54 und 56 wird der diagnostische Modul 10 schließlich beidseitig abgesperrt. Die Leitungen 70, 74, 72 und 76 können nun abgenommen werden und man legt den Modul 10 in einen Brutschrank, um die Keime in der Nährlösung zu bebrüten.

Um nach dem Bebrütungsvorgang die Anzucht der Keime einzuleiten, wird der diagnostische Modul dem Brutschrank entnommen und der Deckel 16 (Fig. 1) abgenommen. Nun kann auf sehr einfache Weise die Adsorbensfüllung 28 portionsweise aus dem Gehäuse 12 herausgedrückt und auf entsprechende Agar-Platten oder ein anderes geeignetes Zuchtmedium verteilt werden.

Zu diesem Zweck wird ein Druck, in Fig. 1 von oben, auf das obere Ende der Kolbenstange 22 ausgeübt, so daß sich der Kolben aus der in der Figur dargestellten Position I in der Zeichnung nach unten in Richtung auf die schematisch angedeutete Position II bewegt. Dabei verdrängt er das Adsorbens 28 aus dem durch den Kolben 24 und die Innenwandung 38 des zylinderförmigen Gehäuses 12 begrenzten Raum.

Dieser Vorgang ist in Fig. 3 schematisch dargestellt, wobei das Bezugszeichen 80 eine Petrischale mit einem Agar-Medium bezeichnet. Soweit die Teile des Adsorbens 28 zusammenhaften, können sie, wenn sie aus dem zylinderförmigen Gehäuse 12 herausgedrückt werden, leicht mit einem geeigneten Werkzeug abgelöst und dadurch feindosiert und schnell auf der Petrischale 80 verteilt werden. Diese einfach erscheinende Verbesserung führt zu einem wesentlichen Fortschritt, da es bei der Identifizierung der eine Sepsis verursachenden Keime ganz entscheidend auf die Möglichkeit ankommt, schnell und sauber zu arbeiten.

## Patentansprüche

1. Vorrichtung zum Nachweis von Bakterien, Pilzen und Viren im Blut mit einem Gehäuse (12), in dem sich ein die Bakterien, Pilze und Viren bindendes aus mehreren zur anschließenden bakteriologischen, virologischen, mykologischen oder elektronenmikroskopischen Untersuchung geeigneten Teilen bestehendes Adsorbens (28) befindet, wobei das Gehäuse (12) zwei Anschlüsse (25, 26) für Schlauchleitungen zum Zuführen und Abführen von in einem extrakorporalen Kreislauf fließendem Blut aufweist und wobei das Gehäuse (12) im wesentlichen als Zylinder mit vorzugsweise kreisförmigem Querschnitt ausgebildet ist, dadurch gekennzeichnet, daß ein mittels einer aus dem Gehäuse (12) herausragenden Kolbenstange (22) betätigbarer Kolben (24) zwischen einer ersten Position (I) und einer zweiten Position (II) gleitbar in dem Gehäuse (12) angeordnet ist und daß in der ersten Position (I) durch den Kolben (24), die zylindrische Gehäusewandung (38) und einen abnehmbaren Deckel (16) ein Hohlraum begrenzt wird, in dem sich das Adsorbens (28) während des Anschlusses der Vorrichtung an einen extrakorporalen Kreislauf befindet und daß in der zweiten Position (II) der Hohlraum kleiner ist als in der ersten Position (I), so daß bei der Bewegung des Kolbens (24) von der ersten Position in die zweite Position bei abgenommenen Deckel (16) mindestens ein Teil des Adsorbens (28) aus dem Gehäuse (12) herausgedrückt wird.

2. Vorichtung nach Anspruch 1, dadurch gekennzeichnet, daß einer der Anschlüsse (25) für die blutführenden Schlauchleitungen mit der Kolbenstange (22) verbunden ist, wobei die Kolbenstange (22) eine Längsbohrung (30) aufweist, durch die das Blut strömt.

3. Vorrichtung nach Anspruch 1 und 2, dadurch gekennzeichnet, daß der Kolben (24) derart konisch oder stufenförmig mit geringerem Durchmesser in Richtung Kolbenstange (22) ausgebildet ist, daß beim Verschieben des Kolbens von I in Richtung II ein in der Stufe liegender oder auf dem Konus befindlicher O-Ring irreversibel verrutscht.

4. Vorrichtung nach einem der Ansprüche 1 bis 3, dadurch gekennzeichnet, daß mit beiden Anschlüssen (25, 26) Dreiwegverzeigungen (50, 52) verbunden sind, die mit Absperreinrichtungen (54, 56) versehen sind, derart, daß wahlweise statt dem Blutstrom ein Spülmittelstrom durch das Gehäuse strömen kann.

## Claims

1. Device for the detection of bacteria, fungi and viruses in blood, with a housing (12) in which is present a particulate adsorbent (28) binding bacteria, fungi and viruses suitable for the subsequent bacteriological, virological, mycological or electronmicroscopic investigation, whereby the housing (12) has two connections (25, 26) for tubes for the introduction and removal of blood flowing in an extracorporeal circulation and whereby the housing (12) is essentially constructed as a cylinder with preferably circular cross-section, characterised in that a piston (24) operable by means of a piston rod (22) projecting out of the housing (12) is arranged in the housing (12) so as to be slidable between a first position (I) and a second position (II) and in that, in the first position (I) a hollow space is bounded by the piston (24), the cylindrical housing wall (38) and a removable cover (16) in which hollow space there is present the adsorbent (28) during the connection of the device to an extracorporeal circulation and in that, in the second position (II), the hollow space is smaller than in the first position (I) so that in the case of the movement of the piston (24) from the first position to the second position with the cover (16) removed, at least a part of the adsorbent (28) is forced out of the housing (12).

2. Device according to claim 1, characterised in that one of the connections (25) for the blood-conducting tubes is connected with the piston rod (22) whereby the piston rod (22) has a longitudinal bore (30) through which blood flows.

3. Device according to claim 1 and 2, characterised in that the piston (24) is constructed conically or stepshaped with smaller diameter in the direction of the piston rod (22) in such a manner that, in the case of displacement of the piston from I in the direction II, an O-ring lying in the step or present on the cone slips off irreversibly.

4. Device according to one of Claims 1 to 3, characterised in that with the two connections (25, 26) are connected three-way pieces (50, 52) which are provided with closure devices (54, 56) in such a manner that, as desired, instead of the blood stream, a stream of flushing agent can flow through the housing.

## Revendications

1. Dispositif pour la mise en évidence de bactéries, de champignons et de virus dans le sang, comportant une enveloppe (12), dans laquelle il se trouve un agent adsorbant (28) liant les bactéries, les champigons et les virus, composé de plusieurs parties appropriées à l'examen bactériologique, virologique, mycologique ou par microscopie électronique consécutif, l'enveloppe (12) comportant deux points de branchement (25, 26) pour conduite souples pour l'amenée et l'évacuation de sang circulant dans un circuit extracorporel, l'enveloppe (12) étant réalisée essentiellement sous la forme d'un cylindre ayant de préférence une coupe circulaire, caractérisé en ce qu'un piston (24) actionné au moyen d'un manche (22) s'étendant au dehors de l'enveloppe (12) est disposé dans l'enveloppe (12) de façon à pouvoir être déplacé par glissement entre une première position (I) et une deuxième position (II) et en ce que dans la première position (I) le piston (24), la paroi cylindrique (38) de l'enveloppe et un capuchon (16) amovible délimitant un espace dans lequel se trouve l'agent adsorbant (28) lorsque le dispositif est relié à un circuit extracorporel et en ce que dans la deuxième position (II) l'espace est plus petit que dans la première position (I), de sorte que pendant le mouvement du piston (24) à partir de la première position vers la deuxième position en l'absence du capuchon (16) au moins une partie de l'agent adsorbant (28) est expulsée de l'enveloppe (12).

2. Dispositif selon la revendication 1, caractérisé en ce que l'un des points de branchement (25) pour les conduits souples véhiculant le sang est relié au manche (22) du piston, ce manche (22) du piston présentant un alésage (30) dans le sens de la longueur, dans lequel s'écoule le sang.

3. Dispositif selon les revendications 1 et 2, caractérisé en ce que le piston (24) est réalisé en forme de cône ou à échelon, le diamètre plus faible étant du côté du manche (22) du piston, de telle sorte qu'en déplaçant le piston de I vers II une rondelle torique se trouvant sur l'échelon ou sur le cône change irréversiblement de position.

4. Dispositif suivant l'une quelconque des revendications 1 à 3, caractérisé en ce qu'aux deux points de branchement (25, 26) sont reliés des robinets à trois voies (50, 52), équipés de dispositifs d'obturation (54, 56) de telle façon que, selon le choix, c'est un courant de sang ou d'agent de lavage qui parcourt l'enveloppe.

Fig. 1

Fig. 2

Fig. 3

Fig. 4